# EUROPEAN PATENT APPLICATION

(11) **EP 3 835 409 A1**
(43) Date of publication of application: **16.06.2021**
(21) Application number: 19846840.7
(22) Date of filing: 09.08.2019
(51) Int. Cl.: C12M 3/00, C12N 5/10, C12P 21/08, C07K 16/00

(54) **ALGINATE HOLLOW MICROFIBER**

(30) Priority: 10.08.2018 JP 2018151574
(71) Applicant: Mochida Pharmaceutical Co., Ltd., Tokyo 160-8515 (JP); The University of Tokyo, Bunkyo-ku, Tokyo 113-8654 (JP)
(72) Inventor: FURUSAKO Shoji, Tokyo 160-8515 (JP); TAKEUCHI Shoji, Tokyo 113-8654 (JP)
(74) Representative: Hoffmann Eitle
(86) International application number: PCT/JP2019/031538
(87) International publication number: WO 2020/032221

(57) **Abstract**

Provided are an alginate gel fiber for antibody production wherein an antibody-producing cell (for example, an antibody-producing CHO cell) is comprised in a core layer, and an antibody production method using the alginate gel fiber. An antibody production method is thereby additionally provided.

## Description

### [Technical Field]

The present invention relates to an alginate gel fiber for antibody production, to a method for manufacturing the gel fiber, and to an antibody manufacturing method using the gel fiber.

### [Background Art]

Antibodies are being produced using genetic recombination technology. A variety of antibodies have already been produced using CHO cells, Sp2/0 cells, NSO cells, E. coli or the like as antibody-producing cells.

In particular, CHO cells (derived from Chinese hamster ovaries) are frequently used for culturing antibodies because these cells can be cultured in suspension and have a high growth rate, and because a large quantity of a target protein can be easily produced by large-scale culture of CHO cells.

In recent years, strategies for producing antibody drugs stably at low cost are being sought in the field of antibody drug development and production, and to achieve this, attention has focused on developing more productive and efficient production systems (for example, continuous production methods and novel culture techniques for producing the necessary amount of an antibody using small-scale production equipment).

Antibody-producing cells are being cultured by raising an antibody-producing cell line in a spinner flask or the like, then performing expansion culture while controlling the culture conditions such as the medium composition, temperature, stirring conditions, gas exchange and pH, and finally culturing the cells in a large-scale production culture tank on a scale of thousands to tens of thousands of liters.

When antibody-producing cells are cultured continuously at high densities, the methods for separating the cells from the culture solution and the methods for effectively supplying oxygen and the like may become problems. In the former case, separation methods using gravity settling tubes and continuous centrifuges are being studied, while in the latter case, a variety of methods are being studied such as methods of oxygen diffusion through porous tubes placed in the culture tank and methods of adding fluorocarbons with high oxygen solubility to the culture solution, and various improvements are being devised. However, many problems still need to be resolved in order to efficiently produce antibodies.

Microfibers with a core-shell structure comprising various kinds of cells in the core are known (Patent Literature 1: WO 2011/046105).

Hollow microfibers comprising cell layers and methods for manufacturing such microfibers are also known (Patent Literature 2: WO 2015/178427).

A method is known for preparing a linear cell aggregate by culturing cells that are suspended in an aqueous solution comprising a biocompatible polymer in the hollow part of a alginate gel hollow fiber (Patent Literature 3: Japanese Patent Application Publication No. 2014-236698).

Methods are known for manufacturing and culturing a 3-dimensional cell structure whereby a culture solution can be supplied to every corner of a tissue (Patent Literature 4: Japanese Patent Application Publication No. 2016-77229).

Also known is a microtube provided with a semipermeable membrane comprising cells or a water-soluble chemical substance on the inside and a hydrophobic polymer in the outermost layer (Patent Literature 5: Japanese Patent Application Publication No. 2017-77473).

A method is known for using a microfluidic device having a double coaxial laminar flow to manufacture a meter-long core-shell hydrogel micro fiber encapsulating an extracellular matrix (ECM) protein and differentiated cells or somatic cell stem cells (Non Patent Literature 1).

Also known is a cell-comprising hydrogel micro fiber whereby the mechanical properties and handling properties are improved by using a double network (DN) hydrogel consisted of an alginate and polyacrylamide (Non Patent Literature 2).

However, none of Patent Literature 1 to 5 and Non Patent Literature 1 and 2 either discloses or suggests an alginate gel fiber having high mechanical strength and comprising a core layer comprising antibody-producing cells covered by a shell layer comprising an alginate gel having high mechanical strength (preferably an alginate gel having greater mechanical strength than the core layer), or a method for using this alginate gel fiber to manufacture antibodies.

### [Citation List]

### [Patent Literature]

[Patent Literature 1] WO 2011/046105
[Patent Literature 2] WO 2015/178427
[Patent Literature 3] Japanese Patent Application Publication No. 2014-236698
[Patent Literature 4] Japanese Patent Application Publication No. 2016-77229
[Patent Literature 5] Japanese Patent Application Publication No. 2017-77473

### [Non Patent Literature]

[Non Patent Literature 1] Nature Materials, 12, pp. 584-590, 2013
[Non Patent Literature 2] ACS Biomater. Sci. Eng., 3(3), pp. 392-398, 2017

### [Summary of Invention]

### [Technical Problem]

No alginate gel fiber for antibody production comprising a core layer comprising antibody-producing cells (such as CHO cells) and a base material (such as collagen, medium or an alginic acid solution or alginate gel) covered with a shell layer comprising a crosslinked alginate gel (such as a naturally-derived crosslinked alginate gel) has been known up to now, nor has recombinant antibody production using such a gel fiber. Furthermore, no such alginate gel fiber has been formed with a specific length, nor are methods known for using such an alginate gel fiber to continuously produce antibodies.

Under these circumstances, there is demand for additional antibody production methods.

### [Solution to Problem]

The inventors discovered as a result of earnest research that it was possible to prepare an alginate gel fiber for antibody production comprising a core layer comprising antibody-producing cells and a base material (for example, a base material selected from the group consisting of collagen, medium, an alginic acid solution or alginate gel and the like) covered with a shell layer comprising an alginate gel with high mechanical strength (preferably an alginate gel having greater mechanical strength than the core layer). When an alginate gel fiber thus prepared was used to culture antibody-producing cells, it was discovered that long-term continuous antibody production was possible, thereby perfecting the present invention.

From the Examples below, it was found that (recombinant) antibodies could be produced continuously for a long period of time by preparing an alginate gel fiber with high mechanical strength comprising a core layer comprising a collagen gel as a base material and CHO cells incorporating an antibody gene as antibody-producing cells, covered with a shell layer comprising a calcium-crosslinked alginate gel with high mechanical strength (preferably a calcium-crosslinked alginate gel having greater mechanical strength than the core layer), and culturing this gel fiber. The alginate gel fiber thus prepared provides a suitable environment for antibody-producing CHO cells to continuously produce antibodies, and the antibodies produced in the core layer pass continuously through the shell layer and are released outside the gel fiber.

### [Effect of the Invention]

The present invention further provides an antibody production method.

In some embodiments, the core layer comprising the antibody-producing cells and the base material is covered with a shell layer comprising an alginate gel with high mechanical strength (preferably an alginate gel having greater mechanical strength than the core layer) to provide an alginate gel fiber having high mechanical strength. In the Examples below, an alginate gel fiber is produced by covering a core layer comprising antibody-producing cells with a calcium-crosslinked alginate gel formed from a sodium alginate solution (A-2 or B-2) to form a shell having high mechanical strength, the antibody-producing cells are cultured to continuously produce antibodies over a long period of time in the core layer, and the antibodies then pass through the shell layer and are released continuously outside the alginate gel fiber.

An alginate gel fiber of a preferred embodiment provides an environment suited to antibody production. Because the cells are encapsulated in the core layer, there is little physical stress on the antibody-producing cells in the culture solution, and continuous antibody production by the encapsulated antibody-producing cells can be expected in the long term. Thus, such an antibody manufacturing method using an alginate gel fiber can be expected to have dramatically improved antibody production efficiency. For example, unlike suspension culture of antibodies, which requires a large-scale culture tank, antibody production with small-scale production equipment is anticipated. Continuous production techniques for next-generation antibody drugs that are also suited to small scale production of a variety of antibody drug products are also anticipated.

### [Brief Description of Drawings]

[Fig. 1]

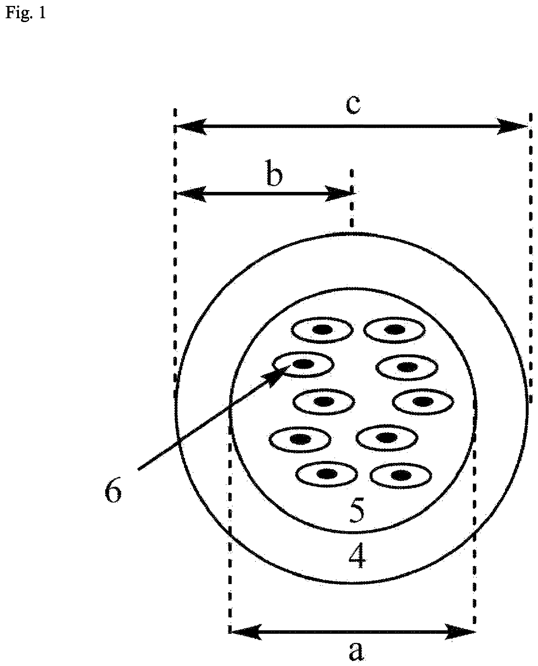

   Fig. 1 is a cross-section of an alginate gel fiber comprising antibody-producing cells in a core layer.
[Fig. 2]

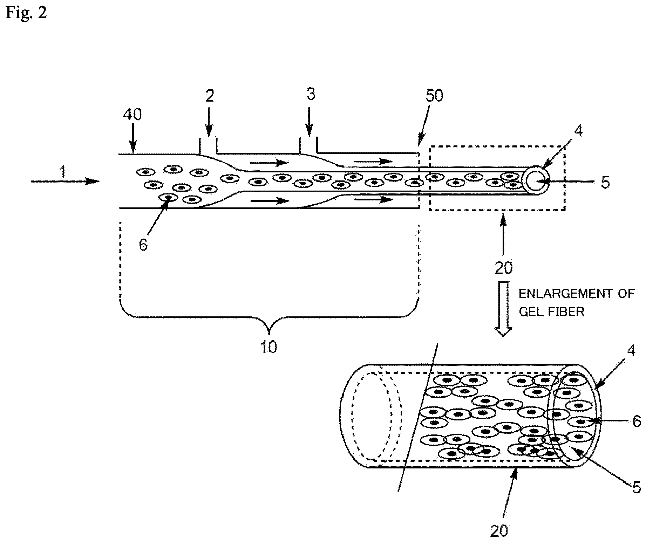

   Fig. 2 is a schematic view explaining one embodiment of a manufacturing process for an alginate gel fiber comprising antibody-producing cells in a core layer.
[Fig. 3]

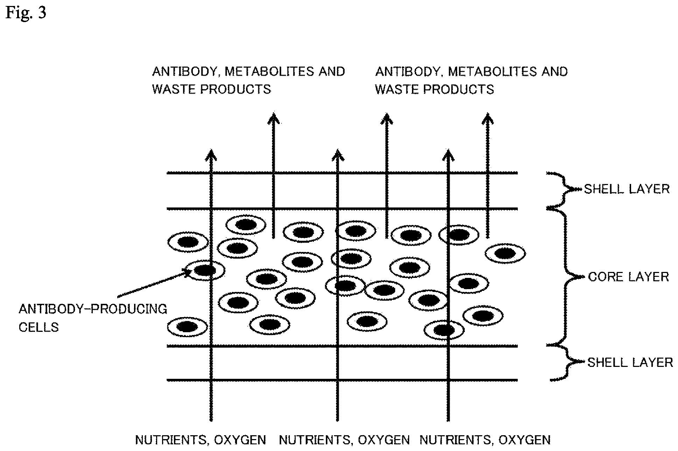

   Fig. 3 is a schematic view showing a horizontal cross-section of an alginate gel fiber comprising antibody-producing cells in a core layer and explaining the passage of the produced antibodies, metabolites, waste products, culture solution (nutrient sources) and oxygen through the shell layer.
[Fig. 4]

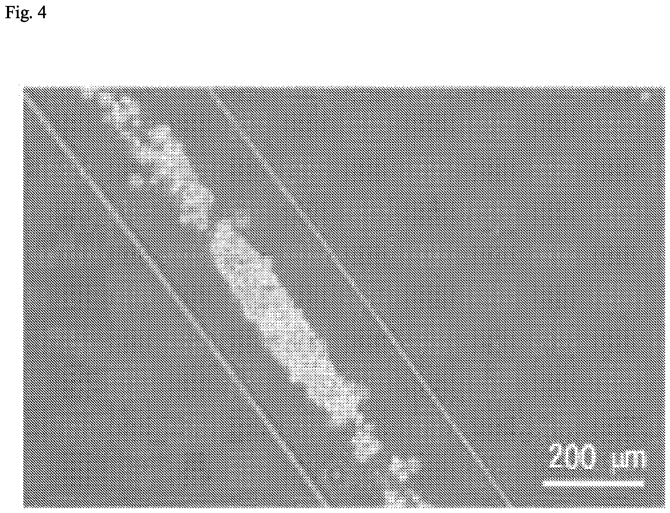

   Fig. 4 is a microscope image of an alginate gel fiber A after culture (12 days).
[Fig. 5]

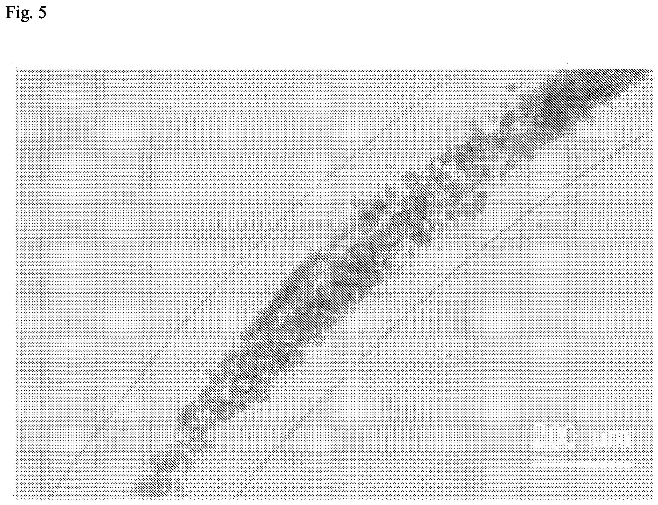

   Fig. 5 is a microscope image of an alginate gel fiber B after culture (28 days).

### [Description of Embodiments]

### [Specific embodiments]

Specific embodiments of the alginate gel fiber, methods for manufacturing the gel fiber and antibody manufacturing methods using the gel fiber are explained here. More specifically, these are the embodiments [1] to [12-1] below.
[1] The first embodiment is an alginate gel fiber formed by covering a core layer comprising antibody-producing cells and a base material with a shell layer comprising an alginate gel having high mechanical strength.
   [1-1] In the Embodiment [1] above, the antibody-producing cells comprised in the core layer are for example cells selected from the group consisting of the CHO cells, CHO cell substrains, COS cells, Sp2/0 cells, NS0 cells, SP2 cells, PERC6 cells, YB2/0 cells, YE2/0 cells, 1R983F cells, Namalwa cells, Wil-2 cells, Jurkat cells, Vera cells, Molt-4 cells, 293-HEK cells, BHK cells, KGH6 cells, P3X63Ag8.653 cells, C127 cells, JC cells, LA7 cells, ZR-45-30 cells, hTERT cells, NM2C5 cells, UACC-812 cells and the like; and preferably are cells selected from the group consisting of the CHO cells, CHO cell substrains, COS cells, Sp2/0 cells, NS0 cells, SP2 cells and PERC6 cells; or more preferably cells selected from the group consisting of the CHO cells, Sp2/0 cells and NS0 cells; or still more preferably CHO cells.
   [1-2] In the Embodiment [1] or [1-1] above, the antibody-producing cells comprised in the core layer of the alginate gel fiber are CHO cells, and for example the CHO cells are CHO cells selected from the group consisting of the muromonab-CD3-producing CHO cells, trastuzumab-producing CHO cells, rituximab-producing CHO cells, palivizumab-producing CHO cells, infliximab-producing CHO cells, basiliximab-producing CHO cells, tocilizumab-producing CHO cells, gemtuzumab ozogamicin-producing CHO cells, bevacizumab-producing CHO cells, ibritumomab tiuxetan-producing CHO cells, adalimumab-producing CHO cells, cetuximab-producing CHO cells, ranibizumab-producing CHO cells, omalizumab-producing CHO cells, eculizumab-producing CHO cells, panitumumab-producing CHO cells, ustekinumab-producing CHO cells, golimumab-producing CHO cells, canakinumab-producing CHO cells, denosumab-producing CHO cells, mogamulizumab-producing CHO cells, certolizumab pegol-producing CHO cells, ofatumumab-producing CHO cells, pertuzumab-producing CHO cells, trastuzumab emtansine-producing CHO cells, brentuximab vedotin-producing CHO cells, natalizumab-producing CHO cells, nivolumab-producing CHO cells, alemtuzumab-producing CHO cells, secukinumab-producing CHO cells, ramucirumab-producing CHO cells, ipilimumab-producing CHO cells, evolocumab-producing CHO cells, mepolizumab-producing CHO cells, alirocumab-producing CHO cells, ixekizumab-producing CHO cells, brodalumab-producing CHO cells, idarucizumab-producing CHO cells, elotuzumab-producing CHO cells, pembrolizumab-producing CHO cells, sarilumab-producing CHO cells, bezlotoxumab-producing CHO cells, belimumab-producing CHO cells, daratumumab-producing CHO cells, avelumab-producing CHO cells, dupilumab-producing CHO cells, atezolizumab-producing CHO cells, benralizumab-producing CHO cells, inotuzumab ozogamicin-producing CHO cells, emicizumab-producing CHO cells, guselkumab-producing CHO cells, durvalumab-producing CHO cells, obinutuzumab-producing CHO cells and vedolizumab-producing CHO cells; or preferably are CHO cells selected from the group consisting of the trastuzumab-producing CHO cells, rituximab-producing CHO cells, infliximab-producing CHO cells, tocilizumab-producing CHO cells, adalimumab-producing CHO cells and nivolumab-producing CHO cells; or more preferably are tocilizumab-producing CHO cells.
   [1-3] In any one of the Embodiments [1] to [1-2] above, the base material comprised in the core layer is for example a base material selected from the group consisting of a collagen solution, a collagen gel, a medium (or culture solution), an alginic acid solution (such as a sodium alginate solution), an alginate gel and mixtures thereof and the like; or preferably is a base material selected from the group consisting of a collagen solution, a collagen gel, a medium (or culture solution), an alginic acid solution and an alginate gel; or more preferably is a collagen solution or a collagen gel.
[2] The second embodiment is the alginate gel fiber according to any one of Embodiments [1] to [1-3] above, wherein the raw material of the alginate gel comprised in the shell layer is sodium alginate, and the M/G ratio of the sodium alginate is in the range of from 0.4 to 1.8 or from 0.1 to 0.4.
[3] The third embodiment is the alginate gel fiber according to Embodiments [1] or [2] above, wherein the raw material of the alginate gel comprised in the shell layer is sodium alginate, and the molecular weight (GPC) of the sodium alginate is in the range of from 700,000 to 1,000,000 or from 800,000 to 1,000,000.
[4] The fourth embodiment is the alginate gel fiber according to any one of Embodiments [1] to [3] above, wherein the raw material of the alginate gel comprised in the shell layer is sodium alginate, and the 1 w/w% viscosity of the sodium alginate is in the range of from 50 to 150 (mPa • s) or from 70 to 150 (mPa • s).
[5] The fifth embodiment is the alginate gel fiber according to any one of Embodiments [1] to [4] above, wherein the outer diameter of the alginate gel fiber is in the range of from 0.2 µm to 2,000 µm for example.
   [5-1] In the Embodiment [5] above, the outer diameter of the alginate gel fiber is in the range of from 50 µm to 1,000 µm for example, or preferably is 300 µm.
[6] The sixth embodiment is the alginate gel fiber according to any one of Embodiments [1] to [5-1] above, wherein the core of the alginate gel fiber has a diameter in the range of from 0.1 µm to 1,000 µm for example.
   [6-1] In the Embodiment [6] above, the core of the alginate gel fiber has a diameter in the range of from 10 µm to 150 µm for example, or preferably is 100 µm.
[7] The seventh embodiment is the alginate gel fiber according to any one of Embodiments [1] to [6-1] above, wherein the outer diameter of the alginate gel fiber is in the range of from 0.2 µm to 2,000 µm and the core layer of the alginate gel fiber has a diameter in the range of from 0.1 µm to 1,000 µm.
   [7-1] In the Embodiment [7] above, for example the outer diameter of the alginate gel fiber is in the range of from 50 µm to 1,000 µm while the core of the alginate gel fiber has a diameter in the range of from 10 µm to 150 µm; or preferably the outer diameter of the alginate gel fiber is 300 µm while the core of the alginate gel fiber has a diameter of 100 µm.
[8] The eighth embodiment is a method for manufacturing an alginate gel fiber with high mechanical strength formed by covering a core layer comprising antibody-producing cells and a base material with a shell layer comprising an alginate gel,
   wherein the alginate gel fiber manufacturing method uses a microfluidic device 10 comprising an introduction pipe 40, an inlet 1 of the introduction pipe 40, an inlet 2 of the introduction pipe 40 located downstream from the inlet 1, an inlet 3 of the introduction pipe 40 located downstream from the inlet 2, and an outlet 50 of the introduction pipe 40 located downstream from the inlet 2, and comprises
   (1) a step of forming a first laminar flow of the antibody-producing cells 6 and the base material in the introduction pipe 40 by introducing antibody-producing cells 6 and a base material from the inlet 1 and injecting the same,
   (2) a step of forming a second laminar flow of the sodium alginate solution covering the outer circumference of the first laminar flow by introducing a sodium alginate solution from the inlet 2 and injecting the same,
   (3) a step of forming a third laminar flow of the solution comprising the divalent metal ion covering the outer circumference of the second laminar flow by introducing a solution comprising a divalent metal ion from the inlet 3 and injecting the same, and
   (4) a step of obtaining an alginate gel fiber formed by covering a core layer comprising antibody-producing cells and a base material that are ejected from the outlet 50 with a shell layer comprising an alginate gel.
      [8-1] In the Embodiment [8] above, the antibody-producing cells 6 are the cells described in Embodiment [1-1] above for example, and namely are for example cells selected from the group consisting of the CHO cells, CHO cell substrains, COS cells, Sp2/0 cells, NS0 cells, SP2 cells, PERC6 cells, YB2/0 cells, YE2/0 cells, 1R983F cells, Namalwa cells, Wil-2 cells, Jurkat cells, Vera cells, Molt-4 cells, 293-HEK cells, BHK cells, KGH6 cells, P3X63Ag8.653 cells, C127 cells, JC cells, LA7 cells, ZR-45-30 cells, hTERT cells, NM2C5 cells, UACC-812 cells and the like; or preferably cells selected from the group consisting of the CHO cells, CHO cell substrains, COS cells, Sp2/0 cells, NS0 cells, SP2 cells and PERC6 cells; or more preferably cells selected from the group consisting of the CHO cells, Sp2/0 cells and NS0 cells; or still more preferably CHO cells.
      [8-2] In the Embodiment [8] or [8-1] above, the antibody-producing cells comprised in the core layer of the alginate gel fiber are CHO cells, and for example the CHO cells are CHO cells selected from the group consisting of the muromonab-CD3-producing CHO cells, trastuzumab-producing CHO cells, rituximab-producing CHO cells, palivizumab-producing CHO cells, infliximab-producing CHO cells, basiliximab-producing CHO cells, tocilizumab-producing CHO cells, gemtuzumab ozogamicin-producing CHO cells, bevacizumab-producing CHO cells, ibritumomab tiuxetan-producing CHO cells, adalimumab-producing CHO cells, cetuximab-producing CHO cells, ranibizumab-producing CHO cells, omalizumab-producing CHO cells, eculizumab-producing CHO cells, panitumumab-producing CHO cells, ustekinumab-producing CHO cells, golimumab-producing CHO cells, canakinumab-producing CHO cells, denosumab-producing CHO cells, mogamulizumab-producing CHO cells, certolizumab pegol-producing CHO cells, ofatumumab-producing CHO cells, pertuzumab-producing CHO cells, trastuzumab emtansine-producing CHO cells, brentuximab vedotin-producing CHO cells, natalizumab-producing CHO cells, nivolumab-producing CHO cells, alemtuzumab-producing CHO cells, secukinumab-producing CHO cells, ramucirumab-producing CHO cells, ipilimumab-producing CHO cells, evolocumab-producing CHO cells, mepolizumab-producing CHO cells, alirocumab-producing CHO cells, ixekizumab-producing CHO cells, brodalumab-producing CHO cells, idarucizumab-producing CHO cells, elotuzumab-producing CHO cells, pembrolizumab-producing CHO cells, sarilumab-producing CHO cells, bezlotoxumab-producing CHO cells, belimumab-producing CHO cells, daratumumab-producing CHO cells, avelumab-producing CHO cells, dupilumab-producing CHO cells, atezolizumab-producing CHO cells, benralizumab-producing CHO cells, inotuzumab ozogamicin-producing CHO cells, emicizumab-producing CHO cells, guselkumab-producing CHO cells, durvalumab-producing CHO cells, obinutuzumab-producing CHO cells and vedolizumab-producing CHO cells; or preferably CHO cells selected from the group consisting of the trastuzumab-producing CHO cells, rituximab-producing CHO cells, infliximab-producing CHO cells, tocilizumab-producing CHO cells, adalimumab-producing CHO cells and nivolumab-producing CHO cells; or more preferably are tocilizumab-producing CHO cells.
      [8-3] In any one of the Embodiments [8] to [8-2] above, the base material comprised in the core layer is for example selected a base material selected from the group consisting of a collagen solution, a collagen gel, a medium (or culture solution), an alginic acid solution (such as a sodium alginate solution), an alginate gel and mixtures thereof and the like; or preferably is a base material selected from the group consisting of a collagen solution, a collagen gel, a medium (or culture solution), an alginic acid solution and an alginate gel; or more preferably is a collagen solution or a collagen gel.
      [8-4] In any one of the Embodiments [8] to [8-3] above, the divalent metal ion is an ion selected from the group consisting of the calcium ions, magnesium ions, barium ions, strontium ions, zinc ions and the like for example; and preferably is a calcium ion.
      [8-5] In any one of the Embodiments [8] to [8-4] above, the solution comprising the divalent metal ion is an aqueous solution selected from the group consisting of a calcium chloride aqueous solution, a calcium carbonate aqueous solution, a calcium gluconate aqueous solution and the like for example; and preferably is a calcium chloride aqueous solution.
      [8-6] In any one of the Embodiments [8] to [8-5] above, the divalent metal ion concentration of the solution comprising the divalent metal ion is for example in the range of from 1 mM to 1 M, or in the range of 50 to 500 mM; or preferably is 100 mM.
      [8-7] In any one of the Embodiments [8] to [8-6] above, the flow rate of the antibody-producing cells 6 and base material injected from the inlet 1 of the microfluidic device 10 is in the range of from 10 to 500 µl/minute for example.
      [8-8] In any one of the Embodiments [8] to [8-7] above, the flow rate of the sodium alginate solution injected from the inlet 2 of the microfluidic device 10 is in the range of from 10 to 500 µl/minute for example.
      [8-9] In any one of the Embodiments [8] to [8-8] above, the flow rate of the solution comprising a divalent metal ion that is injected from the inlet 3 of the microfluidic device 10 is in the range of from 1 to 10 ml/minute for example.
      [8-10] In any one of the Embodiments [8] to [8-9] above, for example the flow rate of the antibody-producing cells 6 and base material injected from the inlet 1 of the microfluidic device 10 is in the range of from 10 to 500 µl/minute, the flow rate of the sodium alginate solution injected from the inlet 2 of the microfluidic device 10 is in the range of from 10 to 500 µl/minute, and the flow rate of the solution comprising a divalent metal ion that is injected from the inlet 3 of the microfluidic device 10 is in the range of from 1 to 10 ml/minute.
      [8-11] In any one of the Embodiments [8] to [8-10] above, the temperature during manufacture of the alginate gel fiber is in the range of from 4°C to 25°C for example.
[9] The ninth embodiment is a method for manufacturing an antibody using an alginate gel fiber formed by covering a core layer comprising antibody-producing cells and a base material with a shell layer comprising an alginate gel, wherein an alginate gel fiber according to any one of the Embodiments [1] to [7-1] above is placed in a culture vessel, medium is added to impregnate the alginate gel fiber, and shaking culture is performed to produce the antibody.
   [9-1] In the Embodiment [9] above, the culture vessel is for example a vessel selected from the group consisting of a triangular flask, a T-flask and a spinner flask, and is preferably a triangular flask.
   [9-2] In the Embodiment [9] or [9-1] above, the conditions for shaking culture are for example a temperature of 37°C and a speed of 125 rpm in a 5% CO₂ incubator.
   [9-3] In any one of the Embodiments [9] to [9-2] above, the period of shaking culture is 30 days for example, or 12 days, or 28 days.
   [9-4] In any one of the Embodiments [9] to [9-3] above, the antibody-producing cells, are for example the cells described in Embodiment [1-1] above, namely for example cells selected from the group consisting of the CHO cells, CHO cell substrains, COS cells, Sp2/0 cells, NS0 cells, SP2 cells, PERC6 cells, YB2/0 cells, YE2/0 cells, 1R983F cells, Namalwa cells, Wil-2 cells, Jurkat cells, Vera cells, Molt-4 cells, 293-HEK cells, BHK cells, KGH6 cells, P3X63Ag8.653 cells, C127 cells, JC cells, LA7 cells, ZR-45-30 cells, hTERT cells, NM2C5 cells, UACC-812 cells and the like; or preferably cells selected from the group consisting of the CHO cells, CHO cell substrains, COS cells, Sp2/0 cells, NS0 cells, SP2 cells and PERC6 cells; or more preferably cells selected from the group consisting of the CHO cells, Sp2/0 cells and NS0 cells; or still more preferably CHO cells.
   [9-5] In any one of the Embodiments [9] to [9-4] above, the antibody-producing cells are CHO cells, and the CHO cells are for example CHO cells selected from the group consisting of the muromonab-CD3-producing CHO cells, trastuzumab-producing CHO cells, rituximab-producing CHO cells, palivizumab-producing CHO cells, infliximab-producing CHO cells, basiliximab-producing CHO cells, tocilizumab-producing CHO cells, gemtuzumab ozogamicin-producing CHO cells, bevacizumab-producing CHO cells, ibritumomab tiuxetan-producing CHO cells, adalimumab-producing CHO cells, cetuximab-producing CHO cells, ranibizumab-producing CHO cells, omalizumab-producing CHO cells, eculizumab-producing CHO cells, panitumumab-producing CHO cells, ustekinumab-producing CHO cells, golimumab-producing CHO cells, canakinumab-producing CHO cells, denosumab-producing CHO cells, mogamulizumab-producing CHO cells, certolizumab pegol-producing CHO cells, ofatumumab-producing CHO cells, pertuzumab-producing CHO cells, trastuzumab emtansine -producing CHO cells, brentuximab vedotin-producing CHO cells, natalizumab-producing CHO cells, nivolumab-producing CHO cells, alemtuzumab-producing CHO cells, secukinumab-producing CHO cells, ramucirumab-producing CHO cells, ipilimumab-producing CHO cells, evolocumab-producing CHO cells, mepolizumab-producing CHO cells, alirocumab-producing CHO cells, ixekizumab-producing CHO cells, brodalumab-producing CHO cells, idarucizumab-producing CHO cells, elotuzumab-producing CHO cells, pembrolizumab-producing CHO cells, sarilumab-producing CHO cells, bezlotoxumab-producing CHO cells, belimumab-producing CHO cells, daratumumab-producing CHO cells, avelumab-producing CHO cells, dupilumab-producing CHO cells, atezolizumab-producing CHO cells, benralizumab-producing CHO cells, inotuzumab ozogamicin-producing CHO cells, emicizumab-producing CHO cells, guselkumab-producing CHO cells, durvalumab-producing CHO cells, obinutuzumab-producing CHO cells and vedolizumab-producing CHO cells; or preferably CHO cells selected from the group consisting of the trastuzumab-producing CHO cells, rituximab-producing CHO cells, infliximab-producing CHO cells, tocilizumab-producing CHO cells, adalimumab-producing CHO cells and nivolumab-producing CHO cells; or more preferably tocilizumab-producing CHO cells.
[10] In the tenth embodiment, an antibody that is produced in the core layer and passes through the shell layer of an alginate gel fiber obtained by an antibody manufacturing method according to any one of the Embodiments [9] to [9-5] above is an antibody having an isotype selected from the group consisting of IgG, IgA, IgM, IgD, IgE and the like.
   [10-1] In the Embodiment [10] above, the isotype of the antibody that is produced in the core layer and passes through the shell layer is preferably IgG or IgE, and more preferably is IgG.
[11] In the eleventh embodiment, the molecular weight of the antibody that is produced in the core layer and passes through the shell layer of an alginate gel fiber obtained by an antibody manufacturing method according to any one of the Embodiments [9] to [9-5] above is in the range of from 45,000 to 900,000.
   [11-1] In the Embodiment [11] above, the molecular weight of the antibody that is produced in the core layer and passes through the shell layer is in the range of preferably from 45,000 to 160,000, or more preferably from 140,000 to 150,000.
[12] In the twelfth embodiment, the antibody that is produced by an antibody manufacturing method according to any one of the Embodiments [9] to [9-5] above is for example muromonab-CD3 from muromonab-CD3-producing CHO cells, trastuzumab from trastuzumab-producing CHO cells, rituximab from rituximab-producing CHO cells, palivizumab from palivizumab-producing CHO cells, infliximab from infliximab-producing CHO cells, basiliximab from basiliximab-producing CHO cells, tocilizumab from tocilizumab-producing CHO cells, gemtuzumab ozogamicin from gemtuzumab ozogamicin-producing CHO cells, bevacizumab from bevacizumab-producing CHO cells, ibritumomab tiuxetan from ibritumomab tiuxetan-producing CHO cells, adalimumab from adalimumab-producing CHO cells, cetuximab from cetuximab-producing CHO cells, ranibizumab from ranibizumab-producing CHO cells, omalizumab from omalizumab-producing CHO cells, eculizumab from eculizumab-producing CHO cells, panitumumab from panitumumab-producing CHO cells, ustekinumab from ustekinumab-producing CHO cells, golimumab from golimumab-producing CHO cells, canakinumab from canakinumab-producing CHO cells, denosumab from denosumab-producing CHO cells, mogamulizumab from mogamulizumab-producing CHO cells, certolizumab pegol from certolizumab pegol-producing CHO cells, ofatumumab from ofatumumab-producing CHO cells, pertuzumab from pertuzumab-producing CHO cells, trastuzumab emtansine from trastuzumab emtansine-producing CHO cells, brentuximab vedotin from brentuximab vedotin-producing CHO cells, natalizumab from natalizumab-producing CHO cells, nivolumab from nivolumab-producing CHO cells, alemtuzumab from alemtuzumab-producing CHO cells, secukinumab from secukinumab-producing CHO cells, ramucirumab from ramucirumab-producing CHO cells, ipilimumab from ipilimumab-producing CHO cells, evolocumab from evolocumab-producing CHO cells, mepolizumab from mepolizumab-producing CHO cells, alirocumab from alirocumab-producing CHO cells, ixekizumab from ixekizumab-producing CHO cells, brodalumab from brodalumab-producing CHO cells, idarucizumab from idarucizumab-producing CHO cells, elotuzumab from elotuzumab-producing CHO cells, pembrolizumab from pembrolizumab-producing CHO cells, sarilumab from sarilumab-producing CHO cells, bezlotoxumab from bezlotoxumab-producing CHO cells, belimumab from belimumab-producing CHO cells, daratumumab from daratumumab-producing CHO cells, avelumab from avelumab-producing CHO cells, dupilumab from dupilumab-producing CHO cells, ateozolizumab from atezolizumab-producing CHO cells, benralizumab from benralizumab-producing CHO cells, inotuzumab ozogamicin from inotuzumab ozogamicin-producing CHO cells, emicizumab from emicizumab-producing CHO cells, guselkumab from guselkumab-producing CHO cells, durvalumab from durvalumab-producing CHO cells, obinutuzumab from obinutuzumab-producing CHO cells or vedolizumab from vedolizumab-producing CHO cells.
   [12-1] In Embodiment [12], the antibody that can be produced by an antibody manufacturing method according to any one of Embodiments [9] to [9-5] above is preferably trastuzumab from trastuzumab-producing CHO cells, rituximab from rituximab-producing CHO cells, infliximab from infliximab-producing CHO cells, tocilizumab from tocilizumab-producing CHO cells, adalimumab from adalimumab-producing CHO cells or nivolumab from nivolumab-producing CHO cells; or more preferably tocilizumab from tocilizumab-producing CHO cells.

The details are explained below.

### 1. Alginic acid

In the present Description, references to alginic acid refer to at least one kind of alginic acid (sometimes called "alginic acids") selected from the group consisting of alginic acid, alginic acid esters and salts thereof (such as sodium alginate). The alginic acid used may be either naturally derived or synthetic, but a naturally derived alginic acid is preferred. A preferred alginic acid(s) is a bioabsorbable polysaccharide that is extracted from natural brown algae such as Lessonia, Macrocystis, Laminaria, Ascophyllum, Durvillea, Ecklonia cava, Eisenia bicyclis and Saccharina japonica, and is a polymer obtained by linear polymerization of two kinds of uronic acid, D-mannuronic acid (M) and L-guluronic acid (G). More specifically, this is a block copolymer including a homopolymer fraction of D-mannuronic acid (MM fraction), a homopolymer fraction of L-guluronic acid (GG fraction), and a fraction of randomly arranged D-mannuronic acid and L-guluronic acid (M/G fraction) in arbitrary combination.

Commercial sodium alginate may be used for the sodium alginate. For example, the sodium alginates listed as A-1, A-2, A-3, B-1, B-2 and B-3 in the table below (sold by Mochida Pharmaceutical Co., Ltd) may be used. Table 1 shows the viscosity, weight-average molecular weight and M/G ratio of a 1 w/w% (mass%) aqueous solution of each sodium alginate.

**[Table 1]**

| Sodium alginate | 1 w/w% viscosity (mPa·s) | Weight-average molecular weight | | M/G ratio |
|---|---|---|---|---|
| | | GPC | GPC-MALS | |
| A-1 | 10 to 40 | 300,000 to 400,000 | 60,000 to 80,000 | 0.4 to 1.8 |
| A-2 | 50 to 150 | 700,000 to 1,000,000 | 100,000 to 200,000 | |
| A-3 | 300 to 600 | 1,100,000 to 1,700,000 | 200,000 to 400,000 | |
| B-1 | 10 to 40 | 400,000 to 500,000 | 70,000 to 90,000 | 0.1 to 0.4 |
| B-2 | 70 to 150 | 800,000 to 1,000,000 | 100,000 to 200,000 | |
| B-3 | 400 to 600 | 1,500,000 to 1,900,000 | 200,000 to 350,000 | |

The physical property values for the sodium alginates A-1, A-2 and A-3 were measured by the methods described below, but the measurement methods are not limited to these.

### [Measuring viscosity of sodium alginate]

This was measured by rotational viscometry (using a cone plate rotational viscometer) following the viscosity measurement methods of the Japanese Pharmacopoeia (16th Edition). The specific measurement conditions are as follows. The sample solution was prepared using MilliQ water. A cone plate rotational viscometer (RS600 RheoStress rheometer (Thermo Haake GmbH), sensor: 35/1) was used as the measurement equipment. The rotation was set at 1 rpm when measuring a 1 w/w% (mass%) sodium alginate solution. For the read time, the measurement was performed for 2 minutes and the average value from 1 to 2 minutes after starting was used. The average of three measured values was used as the measurement value. The measurement temperature was 20°C.

### [Measuring weight-average molecular weight of sodium alginate]

This was measured by two measurement methods, (1) gel permeation chromatography (GPC) and (2) GPC-MALS. The measurement conditions are as follows.

### [Pre-treatment methods]

An eluent was added to dissolve the sample, which was then filtered through an 0.45-micron membrane filter to obtain a measurement solution.

### (1) Gel permeation chromatography (GPC) measurement

### [Measurement conditions (relative molecular weight distribution measurement)]

Columns: TSKgel GMPW-XL×2+G2500PW-XL (7.8 mm I.D.x300 mmx3)
Eluent: 200 mM sodium nitrate aqueous solution
Flow rate: 1.0 ml/min
Concentration: 0.05%
Detector: RI detector
Column temperature: 40°C
Injection volume: 200 µl
Molecular weight standards: Standard pullulan, glucose

### (2) GPC-MALS measurement

### [Refractive index increment (dn/dc) measurement (measurement conditions)]

Differential refractometer: Optilab T-rEX
Measurement wavelength: 658 nm
Measurement temperature: 40°C
Solvent: 200 mM sodium nitrate aqueous solution
Sample concentrations: 0.5 to 2.5 mg/ml (5 concentrations)

### [Measurement conditions (absolute molecular weight distribution measurement)]

Columns: TSKgel GMPW-XL×2+G2500PW-XL (7.8 mm I.D.×300 mm×3)
Eluent: 200 mM sodium nitrate aqueous solution
Flow rate: 1.0 ml/min
Concentration: 0.05%
Detectors: RI detector, light scattering detector (MALS)
Column temperature: 40°C
Injection volume: 200 µl

In this Description, the molecular weights of alginic acid, alginic acid derivatives, crosslinked alginic acid and crosslinked alginic acid may be given in units of Da (Daltons).

An alginic acid's constituent ratio of D-mannuronic acid to L-guluronic acid (M/G ratio) differs mainly according to the type of seaweed or the like from which it is derived, and may also be affected by the organism's habitat and season, with a wide range from high-G alginic acid (M/G ratio about 0.2) to high-M alginic acid (M/G ratio about 5). The gelling ability of the alginic acids and the properties of the resulting gel are affected by the M/G ratio, and in general, the gel strength of an ion-crosslinked alginate gel is known to be greater the higher the G proportion. The M/G ratio also affects the hardness, fragility, water absorption, flexibility and the like of the gel. The M/G ratio of the alginic acids and/or salt thereof used is for example in the range of from 0.4 to 1.8, or in the range of 0.1 to 0.4.

When numerical ranges are indicated with "from" and "to" this Description, the numbers after "from" and "to" are the minimum and maximum values of the range, respectively.

An "alginic acid ester" or "alginic acid salt" used here is not particularly limited, but because it will react with a crosslinking agent, it must have no functional groups that would impede the crosslinking reaction. Desirable examples of alginic acid esters include propylene glycol alginate and the like.

Examples of alginic acid salts include monovalent salts and divalent salts of alginic acid. Preferred examples of monovalent alginic acid salts include sodium alginate, potassium alginate and ammonium alginate, of which sodium alginate and potassium alginate are more preferred, and sodium alginate is especially preferred. Preferred examples of divalent alginic acid salts include calcium alginate, magnesium alginate, barium alginate and strontium alginate and the like.

Alginic acids are high-molecular-weight polysaccharides, and their molecular weights are hard to determine accurately, but generally the weight-average molecular weight is in the range of 1,000 to 10,000,000, or preferably 10,000 to 8,000,000, or more preferably 20,000 to 3,000,000. It is known that in molecular weight measurement of naturally derived high-molecular-weight substances, values may differ depending on the measurement method.

In certain embodiments, the weight-average molecular weight of the alginic acid as measured by gel permeation chromatography (GPC) or gel filtration chromatography (which together are also called size exclusion chromatography) is for example in the range of from 300,000 to 400,000, or in the range of from 700,000 to 1,000,000, or in the range of from 1,100,000 to 1,700,000, or in the range of from 400,000 to 500,000, or in the range of from 800,000 to 1,000,000, or in the range of from 1,500,000 to 1,900,000.

For example, the weight-average molecular weight as measured by gel permeation chromatography (GPC) or gel filtration chromatography (which together are also called size exclusion chromatography) is in the range of from 300,000 to 400,000 in the case of sodium alginate A-1; in the range of from 700,000 to 1,000,000 in the case of sodium alginate A-2; in the range of from 1,100,000 to 1,700,000 in the case of sodium alginate A-3; in the range of from 400,000 to 500,000 in the case of sodium alginate B-1; in the range of from 800,000 to 1,000,000 in the case of sodium alginate B-2; and in the range of from 1,500,000 to 1,900,000 in the case of sodium alginate B-3.

In a preferred embodiment, the weight-average molecular weight of the sodium alginate as measured by gel permeation chromatography (GPC) or gel filtration chromatography (which together are also called size exclusion chromatography) is in the range of from 700,000 to 1,000,000, or in the range of from 800,000 to 1,000,000.

Preferably it is in the range of 700,000 to 1,000,000 in the case of the sodium alginate A-2, or in the range of from 800,000 to 1,000,000 in the case of the sodium alginate B-2.

The absolute weight-average molecular weight can also be measured by the GPC-MALS method.

In some embodiments, the weight-average molecular weight (absolute molecular weight) as measured by the GPC-MALS method is for example in the range of from 60,000 to 80,000, or in the range of from 100,000 to 200,000, or in the range of from 200,000 to 400,000, or in the range of from 70,000 to 90,000, or in the range of from 100,000 to 200,000, or in the range of from 200,000 to 350,000.

For example, the weight-average molecular weight (absolute molecular weight) as measured by GPC-MALS is in the range of from 60,000 to 80,000 in the case of the sodium alginate A-1; from 100,000 to 200,000 in the case of the sodium alginate A-2; from 200,000 to 400,000 in the case of the sodium alginate A-3; from 70,000 to 90,000 in the case of the sodium alginate B-1; from 100,000 to 200,000 in the case of the sodium alginate B-2; and from 200,000 to 350,000 in the case of the sodium alginate B-3.

In a preferred embodiment, the weight-average molecular weight (absolute molecular weight) as measured by the GPC-MALS is in the range of from 100,000 to 200,000.

Preferably, it is in the range of from 100,000 to 200,000 in the case of the sodium alginate A-2, or in the range of from 100,000 to 200,000 in the case of the sodium alginate B-2.

When the molecular weight of a high-molecular-weight polysaccharide is measured by such methods, a measurement error of 10% to 20% is normal. Thus, for example, a value of 400,000 may vary in the range of from 320,000 to 480,000, a value of 500,000 may vary in the range of 400,000 to 600,000, and a value of 1,000,000 may vary in the range of 800,000 to 1,200,000.

The molecular weight of the alginic acids may be measured by ordinary methods.

Typical conditions for molecular weight measurement using gel filtration chromatography are a described above. A Superose 6 Increase 10/300 GL column (GE Health Care Sciences) may be used as the column, a 10 mmol/L phosphate buffer (pH 7.4) comprising 0.15 mol/L NaCl may be used as the development solvent for example, and blue dextran, thyroglobulin, ferritin, aldolase, conalbumin, ovalbumin, ribonuclease A and aprotinin may be used as molecular weight standards.

The viscosity of the alginic acid used in this Description is not particularly limited, but when measured in a 1 w/w% aqueous solution of the alginic acids, it is preferably from 10 to 40 mPa·s in the case of the sodium alginate A-1; from 50 to 150 mPa·s in the case of the sodium alginate A-2; from 300 to 600 mPa·s in the case of the sodium alginate A-3; from 10 to 40 mPa·s in the case of the sodium alginate B-1; from 70 to 150 mPa·s in the case of the sodium alginate B-2; and from 400 to 600 mPa·s in the case of the sodium alginate B-3.

Preferably it is in the range of from 50 to 150 mPa·s in the case of the sodium alginate A-2 or in the range of from 70 to 150 mPa·s in the case of the sodium alginate B-2.

The viscosity of an aqueous solution of the alginic acid can be measured by ordinary methods. For example, it can be measured by rotational viscometry using a coaxial double cylindrical rotational viscometer, single cylindrical rotary viscometer (Brookfield viscometer), conical plate rotational viscometer (cone plate viscometer) or the like. Preferably it is measured following the viscosity measurement methods of the Japanese Pharmacopoeia (16th Edition). More preferably, a cone plate viscometer is used.

When first extracted from brown algae, alginic acids have high molecular weight and somewhat high viscosity, but the molecular weight and viscosity are reduced by the processes of heat drying, purification and the like. Alginic acids with different molecular weights can be manufactured by methods such as controlling the temperature and other conditions during the manufacturing process, selecting the brown algae used as raw materials, and fractioning the molecular weights in the manufacturing process. Alginic acids having the desired molecular weight can also be obtained by mixing alginic acids from different lots having different molecular weights or viscosities.

In certain other embodiments, the alginic acid (sodium alginate) used to form the core layer or shell layer of the alginate gel fiber is not particularly limited, but may be selected from the sodium alginates A-1, A-2, A-3, B-1, B-2 and B-3 listed in Table 1 above for example. The concentration of a sodium alginate solution prepared using one of these sodium alginates is in the range of from 0.1 to 2.0 mass% (w/w%) for example. Furthermore, the sodium alginate used to form the shell layer is preferably A-2 or B-2, and the concentration of a sodium alginate solution prepared using one of these sodium alginates is preferably 1.5 mass% (w/w%).

In certain embodiments, the alginic acid (sodium alginate) used to form the core layer or shell layer of the alginate gel fiber is not particularly limited, but may be used mixed with a collagen solution, medium (or culture solution) or the like.

The solvents used in the sodium alginate solution, collagen solution and the like are as described below.

### 2. Alginate gel (shell layer)

The alginic acid solution is partially crosslinked with a divalent metal ion to form an alginate gel (ion-crosslinked alginic acid).

When the alginic acid contacts a divalent metal ion, the time taken to form the alginate gel is seconds (such as 1 to 5 seconds) to hours (such as 1 to 3 hours).

The divalent metal ion used to obtain the alginate gel is not particularly limited, but examples include calcium ions, magnesium ions, barium ions, strontium ions, zinc ions and the like, and a calcium ion is preferred.

The solution comprising the divalent metal ion is not particularly limited but may be a solution comprising a calcium ion (such as a calcium chloride aqueous solution, calcium carbonate aqueous solution, calcium gluconate aqueous solution or the like) for example and is preferably a calcium chloride aqueous solution.

The divalent ion concentration (such as the calcium ion concentration) of the solution comprising the divalent metal ion is not particularly limited but may be in the range of 1 mM to 1 M or 5 mM to 500 mM for example and is preferably 100 mM.

As discussed above, the physical properties such as the gel strength of the alginic acid used in this method may differ depending on the molecular weight and M/G ratio and the alginic acid concentration and calcium ion concentration of the solution. Consequently, a desirable gel having a high mechanical strength, such as a desirable gel having a greater mechanical strength than the core layer, can be manufactured by adjusting these variables.

The solvents used in the alginic acid solution of the shell layer and the solution comprising the divalent metal ion and the like are not particularly limited, and each may independently be tap water, pure water (such as distilled water, deionized water, RO water or RO-EDI water), ultrapure water (MilliQ water), medium (that is, cell culture medium (or culture solution)), phosphate-buffered saline (PBS), physiological saline or the like, with ultrapure water being preferred.

### 3. Alginate gel fiber

"Alginate gel fiber" means a fibrous structure comprising a core layer and a shell layer comprising an alginate gel. Fig. 1 shows a cross-section of one example of an alginate gel fiber formed as a fiber having a core-shell structure. This alginate gel fiber has an external diameter c, a core layer 5 with a diameter a and a shell layer 4 with a thickness c, and the core layer 5 comprises antibody-producing cells 6 and a base material, while the shell layer 4 comprises an alginate gel.

The base material comprised in the core layer of the alginate gel fiber and the material constituting the shell layer (that is, the alginate gel) may be the same material or different materials. Certain embodiments of the alginate gel fiber are formed by covering a core layer comprising antibody-producing cells and collagen (solvent or gel) with a shell layer comprising an alginate gel, meaning that the base material comprised in the core layer is different from the material constituting the shell layer.

Because the "alginate gel fiber" is a fibrous structure having the above core-shell structure (hollow partial structure passing through central axis) and the external diameter of the alginate gel fiber is about 0.2 µm to 2,000 µm (although this external diameter is not limited), it is sometimes called an "alginate hollow microfiber".

The shape of the alginate gel fiber in a cross-section cut perpendicular to the central axis of the fiber may be a variety of shapes such as circular, oval or polygonal (such as square, pentagonal or the like), but a circular cross-sectional shape such as that shown in Fig. 1 is preferred.

The diameter of the core layer (hollow part) of the alginate gel fiber is not particularly limited but may be in the range of from 0.1 µm to 1,000 µm or from 10 µm to 150 µm for example, or preferably is 100 µm. The inner diameter (bore) of the shell layer is the same as the diameter of the core layer.

The thickness of the shell layer of the alginate gel fiber can be determined by subtracting the diameter of the core layer from the external diameter of the alginate gel fiber, and dividing by 2.

The external diameter of the alginate gel fiber is not particularly limited but may be in the range of from 0.2 µm to 2,000 µm or in the range of from 50 µm to 1,000 µm for example and is preferably 300 µm.

In certain embodiments, the diameter of the core layer of the alginate gel fiber is 100 µm and the external diameter of the alginate gel fiber is 300 µm. In this case, the thickness of the shell layer is 100 µm.

The diameter of the core layer (hollow part) of the alginate gel fiber, the inner diameter of the shell layer and the outer diameter of the alginate gel fiber are measured in images taken with a phase contrast optical microscope and represented as average values of measurements taken at multiple locations. The core layer and shell layer of the alginate gel fiber normally have effectively uniform thicknesses, and preferably the thickness of each layer is uniform within a range of ±5%.

The length of the alginate gel fiber is not particularly limited, and may be from 1 mm to 200 m for example, or from 1 cm to 50 m for example, or preferably from 1 to 30 m.

In this Description, the base material forming the core layer of the alginate gel fiber particularly limited as long as it has no cell toxicity, but may be for example a base material selected from the group consisting of a collagen solution, a collagen gel, a medium (or culture solution), an alginic acid solution (such as a sodium alginate solution), an alginate gel and mixtures thereof and the like; or preferably is a base material selected from the group consisting of a collagen solution, a collagen gel, a medium (or culture solution), an alginic acid solution and an alginate gel; or more preferably is a collagen solution or a collagen gel.

In this Description, the core layer is formed by comprising antibody-producing cells in the base material and then making the whole into a solution of a suitable concentration. When using a sodium alginate solution, this is a 0.1 to 2.0 mass% (w/w%) solution for example, or preferably a 1.5 mass% (w/w%) solution, while when using a collagen solution, this is a 0.1 to 2.0 mass% (w/w%) solution for example, or preferably a 0.2 mass% (w/w%) solution.

The solvent used in the base material of the core layer is not particularly limited, but may be tap water, pure water (such as distilled water, deionized water, RO water or RO-EDI water), ultrapure water (MilliQ water), cell culture medium (or culture solution), phosphate-buffered saline (PBS), physiological saline or the like, with ultrapure water being preferred.

In this Description, a commercial medium material or prepared medium or a medium prepared in-house may be used as the cell culture medium. Either a natural medium (such as LB medium, Nutrient Broth (NB) medium, soybean casein digest medium (SCD medium) or the like) or a synthetic medium (a medium that supplements all of nutrients necessary for growth with chemical substances) may be used. The medium is not particularly limited, but may be any basic medium containing components necessary for cell survival and growth (inorganic salts, carbohydrates, hormones, essential amino acids, non-essential amino acids, vitamins, etc.), such as DMEM, Minimum Essential Medium (MEM), RPMI-1640, Basal Medium Eagle (BME), Dulbecco's Modified Eagle's Medium: Nutrient Mixture F-12 (DMEM/F-12), Glasgow Minimum Essential Medium (Glasgow MEM), G016 medium or the like.

This medium may also contain serum. The serum is not particularly limited, but examples include FBS/FCS (Fetal Bovine/Calf Serum), NCS (Newborn Calf Serum), CS (Calf Serum), HS (Horse Serum) and the like. The concentration of the serum in the medium is from 2 mass% to 10 mass% for example.

There are no particular limitations to the antibody-producing cells that can be comprised in the core of the alginate gel fiber, which may selected appropriately from CHO cells, CHO cell substrains, COS cells, Sp2/0 cells, NS0 cells, SP2 cells, PERC6 cells, YB2/0 cells, YE2/0 cells, 1R983F cells, Namalwa cells, Wil-2 cells, Jurkat cells, Vera cells, Molt-4 cells, 293-HEK cells, BHK cells, KGH6 cells, P3X63Ag8.653 cells, C127 cells, JC cells, LA7 cells, ZR-45-30 cells, hTERT cells, NM2C5 cells, UACC-812 cells or the like for example (some of these cells are described in the ATCC cell catalog available from the American Type Culture Collection).

The antibody-producing cells that can be comprised in the core layer of the alginate gel fiber are preferably CHO cells, CHO cell substrains, COS cells, Sp2/0 cells, NS0 cells, SP2 cells or PERC6 cells, or more preferably CHO cells, Sp2/0 cells or NS0 cells, or still more preferably CHO cells.

In this Description, the CHO cells that can be comprised in the core layer of the alginate gel fiber are not particularly limited, but may be for example muromonab-CD3-producing CHO cells, trastuzumab-producing CHO cells, rituximab-producing CHO cells, palivizumab-producing CHO cells, infliximab-producing CHO cells, basiliximab-producing CHO cells, tocilizumab-producing CHO cells, gemtuzumab ozogamicin-producing CHO cells, bevacizumab-producing CHO cells, ibritumomab tiuxetan-producing CHO cells, adalimumab-producing CHO cells, cetuximab-producing CHO cells, ranibizumab-producing CHO cells, omalizumab-producing CHO cells, eculizumab-producing CHO cells, panitumumab-producing CHO cells, ustekinumab-producing CHO cells, golimumab-producing CHO cells, canakinumab-producing CHO cells, denosumab-producing CHO cells, mogamulizumab-producing CHO cells, certolizumab pegol-producing CHO cells, ofatumumab-producing CHO cells, pertuzumab-producing CHO cells, trastuzumab emtansine-producing CHO cells, brentuximab vedotin-producing CHO cells, natalizumab-producing CHO cells, nivolumab-producing CHO cells, alemtuzumab-producing CHO cells, secukinumab-producing CHO cells, ramucirumab-producing CHO cells, ipilimumab-producing CHO cells, evolocumab-producing CHO cells, mepolizumab-producing CHO cells, alirocumab-producing CHO cells, ixekizumab-producing CHO cells, brodalumab-producing CHO cells, idarucizumab-producing CHO cells, elotuzumab-producing CHO cells, pembrolizumab-producing CHO cells, sarilumab-producing CHO cells, bezlotoxumab-producing CHO cells, belimumab-producing CHO cells, daratumumab-producing CHO cells, avelumab-producing CHO cells, dupilumab-producing CHO cells, atezolizumab-producing CHO cells, benralizumab-producing CHO cells, inotuzumab ozogamicin-producing CHO cells, emicizumab-producing CHO cells, guselkumab-producing CHO cells, durvalumab-producing CHO cells, obinutuzumab-producing CHO cells or vedolizumab-producing CHO cells or the like.

In this Description, the CHO cells that can be comprised in the core layer of the alginate gel fiber are more preferably CHO cells selected from the group consisting of the trastuzumab-producing CHO cells, rituximab-producing CHO cells, infliximab-producing CHO cells, tocilizumab-producing CHO cells, adalimumab-producing CHO cells and nivolumab-producing CHO cells, or still more preferably are tocilizumab-producing CHO cells.

The shell layer of the alginate gel fiber comprises a crosslinked alginate gel (such as a naturally derived crosslinked alginate gel). This alginate gel may be a gel having mechanical strength equivalent to or greater than the underlying core layer comprising the antibody-producing cells and is preferably a gel having greater mechanical strength than the underlying core layer comprising the antibody-producing cells. It is also a gel that is sufficiently permeable to components such as culture solution (nutrients) and oxygen from outside the alginate gel fiber during culture.

The mechanical strength of this crosslinked alginate gel can be measured by measuring the tensile strength, load strength or the like by methods known to those skilled in the art, such as a method using a tensile tester in water. Biological components and non-biological components may also be added to this crosslinked alginate gel as necessary.

In some embodiments, the alginate gel is an alginate gel that is gelled in response to external stimulus. Examples of the external stimulus include, but are not limited to, addition of a divalent metal ion (addition of a solution comprising a divalent metal ion), enzyme treatment, pH change, heating, UV exposure, radiation exposure and the like. A divalent metal ion is preferred.

The divalent metal ion is not particularly limited, but may be a calcium ion, magnesium ion, barium ion, strontium ion, zinc ion or the like for example, and preferably is a calcium ion.

The solution comprising the calcium ion is not particularly limited but may be an aqueous solution such as a calcium chloride aqueous solution, calcium carbonate aqueous solution, calcium gluconate aqueous solution or the like for example, and preferably is a calcium chloride aqueous solution.

Preferably culture can be initiated at an early stage after the gel fiber is formed by infiltrating the alginate gel fiber with the culture solution. More preferably, it is possible to provide a gel fiber having a core layer with a large diameter so as not to cause necrosis of the antibody-producing cells comprised in the core layer. That is, with the alginate gel fiber it is easy to obtain a gel fiber having a core layer comprising a predetermined number of antibody-producing cells.

Depending on the shell layer covering the core layer, there is a risk that the shell layer can collapse or break at the stage of covering the core layer because sufficient mechanical strength is not obtained. However, the inventors discovered that when an alginic acid (such as a naturally-derived alginic acid) is used in the shell layer covering the core layer and cured by crosslinking with a calcium ion, it is possible to obtain an alginate gel fiber with sufficient strength to cover the core layer while allowing culture solution (nutrients) and oxygen to be supplied. In a preferred embodiment, the alginate gel forming the shell layer is an alginate gel having greater mechanical strength than the core layer.

Thus, the alginate gel fiber is an alginate gel fiber formed by covering a core layer comprising antibody-producing cells and a base material with a shell layer comprising an alginate gel having high mechanical strength. The alginate gel fiber here may also be called an "alginate gel fiber formed by covering a core layer comprising antibody-producing cells and a base material with a shell layer comprising a calcium ion crosslinked alginate gel".

The "alginate gel having greater mechanical strength than the core layer" here means an alginate gel whereby there is little risk of collapse or breakage of the shell layer at the stage of covering the core layer because the shell layer of the alginate gel fiber uses a gel (which may an alginate gel or agarose gel, but is not limited to these) having effectively the same or greater mechanical strength than the base material constituting the covered core layer (a base material selected for example from the group consisting of a collagen solution, a collagen gel, a medium (or culture solution), an alginic acid solution (such as a sodium alginate solution), an alginate gel and mixtures thereof and the like).

Preferably an alginate gel fiber having greater mechanical strength than the core layer can be obtained by using an alginate gel having the property of gelling in the presence of metal ions such as calcium ions (for example, a calcium ion crosslinked alginate gel) as the gel forming the shell layer.

The mechanical strength of the gel can be measured by measuring the tensile strength, load strength or the like by methods known to those skilled in the art, such as a method using a tensile tester in water.

The Young's moduli of the gel forming the shell layer and the base material forming the core layer can be compared to verify that the shell layer has greater mechanical strength than the core layer. For example, the Young's modulus of the alginic acid used as one kind of shell layer is 3.6 kPa with a 1 mass% alginic acid or 6.0 kPa with a 2 mass% alginic acid (a 1.5 mass% alginic acid was used in the Examples, and the Young's modulus was estimated to be 3.6 kPa to 6.0 kPa), and given that the collagen used as one base material of the core layer has a Young's modulus of 0.13 kPa, this means that the shell layer has greater mechanical strength than the core layer in an alginate gel fiber in which the shell layer is an alginate gel and the base material forming the core layer is a collagen solution or collagen gel.

The Young's modulus (also called the modulus of elasticity or elastic modulus) is a value defined as the slope of the (linear part of the) elastic range in a stress-strain curve obtained by tensile testing of a material and may be used as an indicator of mechanical strength. For example, the following values are known as the Young's moduli of various materials: 1 % alginic acid: 3.6 kPa, 2 mass% alginic acid: 6.0 kPa, 0.2 mass% agarose: 0.7 kPa, 0.5 mass% agarose: 2.4 kPa, 1 mass% agarose: 3.6 kPa, 2 mass% agarose: 10.6 kPa, 5 mass% PEG-DA (polyethylene glycol diacrylate): 0.5 kPa, 10 mass% PEG-DA: 1.1 kPa, PDMS (polydimethylsiloxane): 1783 kPa, collagen: 0.13 kPa (see Annals of Biomedical Engineering 36(7), pp. 1254-1267, 2008 or Lab Chip 16, pp. 1757-1776, 2016).

The alginate gel fiber may also be a gel fiber sealed at both ends with an alginate gel or the like. Sealing the gel fiber at both ends helps to prevent the core layer from leaking outside the alginate gel fiber during the culture period.

### [Alginate gel fiber manufacturing method]

Provided here is a method for manufacturing an alginate gel fiber formed by covering a core layer comprising antibody-producing cells and a base material with a shell layer comprising an alginate gel, wherein the manufacturing method uses a microfluidic device.

A method for preparing an alginate gel fiber formed by covering a core layer comprising antibody-producing cells and a base material with a shell layer comprising an alginate gel is explained below.

The method for preparing the alginate gel fiber is not particularly limited but is implemented using a microfluidic device. The microfluidic device here is a device that can be used favorably for preparing an alginate gel fiber. Specifically, the microfluidic device is a device with three inlets and one outlet for creating a microchannel, whereby when a first liquid is supplied to the first inlet, a second liquid is supplied to the second inlet and a third liquid is supplied to the third inlet at appropriate speeds, the first and second liquids form a 2-layer clean laminar flow in the channel where the first and second liquids intersect and come together, and downstream from this the first, second and third liquids form a 3-layer clean laminar flow rather than mixing when the first laminar flow intersects the third liquid and the three liquids come together. The microfluidic device may be for example the double coaxial microfluidic device 10 shown in Fig. 2.

Examples of a microfluidic device 10 capable of injecting a core part and a shell part separately so that the two fluids are coaxial are described in detail in Wonje Jeong et al., Hydrodynamic micro fabrication via "on the fly" photopolymerization of microscale fibers and tubes, Lab Chip, 2004, 4, pp. 576-580, Fig. 1, and in Fig. 1 and Fig. 2 of Japanese Patent Application Publication No. 2016-77229 (Applicant: National University Corporation, University of Tokyo). In certain embodiments, an alginate gel fiber can be manufactured using specific examples of the microfluidic device 10 described in this literature or a similar device under similar conditions.

As shown in Fig. 2, the microfluidic device 10 comprises an introduction pipe 40, an inlet 1 of the introduction pipe 40, an inlet 2 of the introduction pipe 40 located downstream from the inlet 1, an inlet 3 of the introduction pipe 40 located downstream from the inlet 2, and an outlet 50 of the introduction pipe 40 located downstream from the inlet 2.

Fig. 2 is a schematic view explaining one embodiment of an alginate gel fiber manufacturing process. A preparation method using a collagen solution for the base material of the core layer and a sodium alginate solution for the base material of the shell is explained here as one example.

The alginate gel fiber can be manufactured for example by a method comprising the following steps (1) to (4):
(1) a step of forming a first laminar flow of the antibody-producing cells 6 and the base material in the introduction pipe 40 by introducing antibody-producing cells 6 and a base material from the inlet 1 and injecting the same,
(2) a step of forming a second laminar flow of the sodium alginate solution covering the outer circumference of the first laminar flow by introducing a sodium alginate solution from the inlet 2 and injecting the same,
(3) a step of forming a third laminar flow of the solution comprising the divalent metal ion covering the outer circumference of the second laminar flow by introducing a solution comprising a divalent metal ion from the inlet 3 and injecting the same, and
(4) a step of obtaining an alginate gel fiber formed by covering a core layer comprising antibody-producing cells and a base material that are ejected from the outlet 50 with a shell layer comprising an alginate gel.

By performing the steps of (1) to (4) above, it is possible to gel the sodium alginate solution of the shell layer and manufacture an alginate gel fiber 20 comprising a calcium-crosslinked alginate gel in the shell layer 4 and comprising antibody-producing cells 6 and a base material in the core layer 5.

The alginate gel fiber obtained by the above method may also be heated for a few minutes (such as 2 to 5 minutes) to about 1 hour at about 37°C for example to thereby gel the collagen solution comprising the antibody-producing cells 6 in the core layer 5.

The injection speeds of the solutions at the inlets 2 and 3 are not particularly limited, but when the inlet 1 of the micro fluidic device 10 is about 50 µm to 2 mm in diameter, the injection speeds may be about 10 to 500 µl/minute for example. The diameter of the core layer and the coating thickness of the shell layer can be adjusted appropriately by adjusting the injection speeds of the solutions at the inlets 2 and 3. For example, the diameter of the core layer and the coating thickness of the shell layer are smaller when the injection speeds of the solutions at the inlets 2 and 3 are increased, and greater when the injection speeds are reduced.

The collagen solution comprising the antibody-producing cells 6 that is injected from the inlet 1 is prepared as follows.

A buffer composed of HBSS, Hepes and NaHCO₃ is added 4:1 to an acidic collagen solution I-PC (Koken Co., Ltd., cat. #IPC-50) to adjust the collagen concentration to 4 mg/ml. This is mixed 1:1 with a cell suspension that has been adjusted to a predetermined concentration with medium to prepare a collagen solution with a final collagen concentration of 2 mg/ml (0.2%) comprising the antibody-producing cells 6.

The flow speed (injection speed) of the antibody-producing cells 6 and base material injected from the inlet 1 of the micro fluidic device 10 is not particularly limited, but may be from 10 to 500 µl/minute for example when the diameter of the micro fluidic device is about 50 µm to 2,000 µm for example.

For the sodium alginate solution injected from the inlet 2, for example a sodium alginate solution with a predetermined concentration (such as 1.5 mass% (w/w%)) can be prepared using the sodium alginate described in Table 1 by adding culture solution (such as CHO culture medium).

The flow rate of the sodium alginate solution injected from the inlet 2 of the micro fluidic device 10 is not particularly limited but may be in the range of about 10 to 500 µl/minute for example when the diameter of the microfluidic device is from 50 µm to 2,000 µm for example.

For the solution comprising a divalent metal ion that is injected from the inlet 3, for example a calcium chloride aqueous solution with a predetermined concentration (such as 100 mM) is prepared using calcium chloride by adding MilliQ water.

The flow rate of the solution comprising a divalent metal ion that is injected from the inlet 3 of the micro fluidic device 10 is not particularly limited but may be in the range of about 1 to 10 ml/minute for example.

The outer diameter of the prepared alginate gel fiber 20 is not particularly limited, but as discussed above, may be in the range of from 0.2 µm to 2,000 µm or from 50 µm to 1,000 µm for example, and preferably is 300 µm. The length of the hollow micro fiber 200 is not particularly limited, but as discussed above, may be in the range of millimeters to meters for example. As discussed above, the cross-sectional shape of the cell fiber 200 may be circular or oval or a polygonal shape such as a square or pentagon for example.

In certain embodiments, antibody-producing cells can be cultured to produce antibodies by culturing the alginate gel fiber in a culture solution. By properly exchanging the culture solution, it is possible to culture the alginate gel fiber continuously to produce antibodies for several months.

### [Method for culturing antibody-producing cells]

An antibody manufacturing method using an alginate gel fiber formed by covering a core layer comprising antibody-producing cells and a base material with a shell layer comprising an alginate gel is provided here. This "antibody manufacturing method" may also be called a "method for culturing antibody-producing cells".

In a preferred embodiment of the method for culturing antibody-producing cells, culture of the antibody-producing cells is initiated immediately after manufacturing an alginate gel fiber formed by covering a core layer comprising antibody-producing cells and a base material is with a shell layer comprising an alginate gel. It is thus possible to quickly supply culture solution (nutrients) and oxygen to the core layer as shown in Fig. 3. In an especially preferred embodiment, antibodies can be produced while adequately preventing necrosis of the antibody-producing cells in the core layer.

One example of the method for culturing antibody-producing cells is explained in detail below, but this example is not limiting. An alginate gel fiber comprising antibody-producing cells in the core layer is placed in a 125 ml triangular polycarbonate flask, and medium with the composition shown in Table 3 below (30 ml) is added to impregnate the gel fiber, which is then cultured under shaking at 125 rpm in a 5% CO₂ incubator at 37°C. In terms of cell passage, no medium exchange is performed during the culture period, but once every 2 to 3 days 1.8 ml of medium is removed and 1.8 ml of Feed (Irvine Co. JX Feed 003, medium containing high concentration of essential nutrients such as glucose and glutamine consumed during cell culture) is added to maintain the total amount of medium at 30 ml.

In a preferred embodiment of an antibody manufacturing technique using the alginate gel fiber, the antibody-producing cells comprised in the core layer do not proliferate beyond a certain number, which is advantageous because there is less physical stress on the cells, allowing the enclosed antibody-producing cells to produce antibodies continuously over a long period of time.

In an especially preferred embodiment, it may be possible to dramatically improve the antibody production and purification efficiency (for example, in contrast to suspension cultures requiring large-scale culture tanks, the microgel fiber of a preferred embodiment may be used to culture antibodies with small-scale production equipment), and provide a continuous production technology for next-generation antibody drugs suited to manufacturing a variety of different antibody drugs (specifically, antibody drugs and the like) in small quantities.

An antibody (such as tocilizumab) produced by culture may be accumulated in the core layer of the alginate gel fiber, but preferably passes through the shell layer of the alginate gel fiber and accumulates in the culture solution outside the alginate gel fiber. Antibody collection and purification may be performed with reference to the descriptions below.

In a preferred embodiment, as shown in Fig. 3, an antibody produced in the core layer can pass through the shell layer and be released in turn outside the alginate gel fiber, forming a cycle that allows continuous antibody culture. In this case, metabolites and waste products may also be released outside the alginate gel fiber.

In fact, in the examples below it was confirmed that the produced antibody (tocilizumab) accumulated in the core layer and the culture solution when A-2 and B-2 in Table 1 below were used as the sodium alginate used as the raw material of the shell layer.

The culture vessel used to culture the alginate gel fiber formed from an alginate gel covering a core layer comprising antibody-producing cells may be for example a triangular flask, T-flask, spinner flask or the like. Preferably it is a triangular flask, and more preferably a triangular polycarbonate flask.

Antibodies are classified into the classes (isotypes) and subclasses shown in Table 2 below based on differences in the structures of their constant regions.

### Classification of human Ig

**[Table 2]**

| Class (isotype) | Subclass | Ratio (%) in Igs | Molecular weight (approximate) |
|---|---|---|---|
| IgG | IgG1 | 65 | 150,000 |
| | IgG2 | 25 | 150,000 |
| | IgG3 | 7 | 170,000 |
| | IgG4 | 3 | 150,000 |
| IgA | * | 10 to 15 | 320,000 |
| IgM | * | 10 | 900,000 |
| IgD | * | ≤1% | 180,000 |
| IgE | * | ≤0.001 % | 200,000 |

In certain embodiments of the antibody manufacturing method, the antibody that is produced by culturing antibody-producing cells in the core layer of the alginate gel fiber and passes through the shell layer is not particularly limited, but may be an antibody having a class (isotype) selected from the group consisting of IgG, IgA, IgM, IgD, IgE and the like. The antibody that is produced by culturing antibody-producing cells in the core layer of the alginate gel fiber and passes through the shell layer is preferably an IgG or IgE class (isotype) antibody, or more preferably an IgG class (isotype) antibody.

In certain embodiments of the antibody production method, the molecular weight of the antibody that is produced by culturing antibody-producing cells in the core layer of the alginate gel fiber and passes through the shell layer is not particularly limited, but may be in the range of from 45,000 to 900,000 for example. In certain embodiments of the antibody production method, the molecular weight of the antibody that is produced in the core layer of the alginate gel fiber and passes through the shell layer is in the range of preferably from 45,000 to 160,000, or more preferably 140,000 to 150,000.

In this Description, an antibody that corresponds to the antibody-producing CHO cells used is produced when culture is performed by the antibody manufacturing method described above using any of the antibody-producing CHO cells described above. For example, muromonab-CD3 is produced when using muromonab-CD3-producing CHO cells.

Examples of the produced antibody include muromonab-CD3 from muromonab-CD3-producing CHO cells (IgG; 150,000), trastuzumab from trastuzumab-producing CHO cells (IgG; 148,000), rituximab from rituximab-producing CHO cells (IgG; 144,510), palivizumab from palivizumab-producing CHO cells (IgG; 147,700), infliximab from infliximab-producing CHO cells (IgG; 149,000), basiliximab from basiliximab-producing CHO cells (IgG; 147,000), tocilizumab from tocilizumab-producing CHO cells (IgG; 148,000), gemtuzumab ozogamicin from gemtuzumab ozogamicin-producing CHO cells (IgG; 153,000), bevacizumab from bevacizumab-producing CHO cells (IgG; 149,000), ibritumomab tiuxetan from ibritumomab tiuxetan-producing CHO cells (IgG; 148,000), adalimumab from adalimumab-producing CHO cells (IgG; 148,000), cetuximab from cetuximab-producing CHO cells (IgG; 151,800), ranibizumab from ranibizumab-producing CHO cells (IgG; 48,000), omalizumab from omalizumab-producing CHO cells (IgE; 149,000), eculizumab from eculizumab-producing CHO cells (IgG; 145,235), panitumumab from panitumumab-producing CHO cells (IgG; 147,000), ustekinumab from ustekinumab-producing CHO cells (IgG; 148,079 to 149,690), golimumab from golimumab-producing CHO cells (IgG; 149,802 to 151,064), canakinumab from canakinumab-producing CHO cells (IgG; 148,000), denosumab from denosumab-producing CHO cells (IgG; 150,000), mogamulizumab from mogamulizumab-producing CHO cells (IgG; 149,000), certolizumab pegol from certolizumab pegol-producing CHO cells (IgG; 90,000), ofatumumab from ofatumumab-producing CHO cells (IgG; 149,000), pertuzumab from pertuzumab-producing CHO cells (IgG; 148,000), trastuzumab emtansine from trastuzumab emtansine-producing CHO cells (IgG; 151,000), brentuximab vedotin from brentuximab vedotin-producing CHO cells (IgG; 153,000), natalizumab from natalizumab-producing CHO cells (IgG; 146,178), nivolumab from nivolumab-producing CHO cells (IgG; 145,000), alemtuzumab from alemtuzumab-producing CHO cells (IgG; 150,000), secukinumab from secukinumab-producing CHO cells (IgG; 151,000), ramucirumab from ramucirumab-producing CHO cells (IgG; 147,000), ipilimumab from ipilimumab-producing CHO cells (IgG; 148,000), evolocumab from evolocumab-producing CHO cells (IgG; 141,789), mepolizumab from mepolizumab-producing CHO cells (IgG; 149,000), alirocumab from alirocumab-producing CHO cells (IgG; 145,892.049.), ixekizumab from ixekizumab-producing CHO cells (IgG; 149,000), brodalumab from brodalumab-producing CHO cells (IgG; 147,000), idarucizumab from idarucizumab-producing CHO cells (IgG; 47,782), elotuzumab from elotuzumab-producing CHO cells (IgG; 148,000), pembrolizumab from pembrolizumab-producing CHO cells (IgG; 149,000), sarilumab from sarilumab-producing CHO cells (IgG; 150,000), bezlotoxumab from bezlotoxumab-producing CHO cells (IgG; 148,000), belimumab from belimumab-producing CHO cells (IgG; 147,000), daratumumab from daratumumab-producing CHO cells (IgG; 148,000), avelumab from avelumab-producing CHO cells (IgG; 147,000), dupilumab from dupilumab-producing CHO cells (IgG; 152,000), ateozolizumab from atezolizumab-producing CHO cells (IgG; 144,611), benralizumab from benralizumab-producing CHO cells (IgG; 148,000), inotuzumab ozogamicin from inotuzumab ozogamicin-producing CHO cells (IgG; 159,000), emicizumab from emicizumab-producing CHO cells (IgG; 148,000), guselkumab from guselkumab-producing CHO cells (IgG; 146,000), durvalumab from durvalumab-producing CHO cells (IgG; 149,000), obinutuzumab from obinutuzumab-producing CHO cells (IgG; 148,000 to 150,000) or vedolizumab from vedolizumab-producing CHO cells (IgG; 150,000) (the numbers in brackets after the antibody name represent the class (isotype) and molecular weight of each antibody).

The produced antibody is purified for example by the following three steps.
[Step 1] Centrifugation or filtration with a filter or the like is performed to largely remove solid matter and proteins other than the antibody from the medium
[Step 2] The target antibody is extracted by chromatography such as ion-exchange chromatography or affinity chromatography using Protein A or Protein G for example.
[Step 3] Gel filtration chromatography is performed to remove contaminants mixed in in Step 2 and obtain a highly purified target antibody.

### Affinity chromatography using Protein A or Protein G:

Antibody purification methods using Protein A or Protein G for example are known as methods for purifying IgG antibodies. The following method is one example of an antibody purification method using Protein A. (1) Serum is added to a solution obtained by the method of [Step 1] above, and the resulting solution is filtered through a column packed with beads having Protein A fixed thereto, thereby causing the IgG antibody to bind to the beads in the column while the other serum components flow out of the column. (2) An acidic solution in then passed through the column to cleave the IgG antibody bound to the beads, which is then eluted outside the column to obtain the IgG antibody. Since the binding force of Ig antibodies to Protein A and Protein G differs according to the animal species and subclass, Protein A and Protein G can be used separately for different purposes.

### Ion-exchange chromatography:

This is a method for separating proteins based on the electrical properties (charge) of proteins. Since basic proteins exhibiting positive charge bind ionically to negatively charged cation exchangers (carriers) while acidic proteins exhibiting negative charge bind to positively charged anion exchangers, proteins can be bound to an ion exchanger by passing a material containing proteins through a column packed with an ion exchanger. When the salt concentration of the solvent passed through the column is then increased to weaken the ion bonds between the proteins and the ion exchanger, the proteins are released from the ion exchanger and discharged outside the column sequentially starting with the protein having the weakest binding force. The cation exchanger or anion exchanger is selected based on the charges of the proteins used as materials.

### Gel filtration chromatography:

This is a method of separating proteins based on differences in their molecular weights. When a material is passed through a column packed with a porous carrier, proteins having small molecular weights enter and then flow out of the pores, while proteins having high molecular weights flow out of the column without entering the pores, which means that proteins with small molecular weights take more time to pass through the column while proteins with high molecular weights are released more rapidly, and the proteins can be distinguished based on the time difference.

All documents, patent applications, patent publications and other patent literature cited in this Description are herein incorporated by reference. This Description encompasses the matter disclosed in the Claims, Description and drawings of Japanese Patent Application No. 2018-151574 (published August 10, 2018), which is the Japanese patent application on which the priority claim of the present application is based.

### Examples

The present invention is explained below using examples, but the present invention is not limited by these examples.

### [Example 1] Method for preparing alginate gel fiber A

Following the manufacturing steps shown schematically in Fig. 2, an alginate gel fiber A was prepared under the same conditions as the alginate gel fiber described in Japanese Patent Application Publication No. 2016-77229 (Applicant: National University Corporation, University of Tokyo). Tocilizumab-producing CHO cells were used as the cells. An 0.2 mass% collagen solution (5 mg acidic collagen solution I-PC, pH 3.0, sterile Atelocollag, Koken Co., Ltd. Cat. #IPC-50) containing cells at a cell concentration of 1×10⁶ cells/ml (2×10⁵ cells per one 25 m fiber) was introduced through the inlet 1 of the microfluidic device 10, while a 1.5 mass% (w/w%) sodium alginate (A-2, Mochida Pharmaceutical Co., Ltd) solution (solvent: medium with composition of Table 3 below) was introduced from the inlet 2, and 100 mM calcium chloride aqueous solution was introduced and injected through the inlet 3 to obtain an alginate gel fiber A with a core layer diameter of about 100 µm, an outer diameter of about 300 µm and a total length of 25 meters.

### [Example 2] Method for preparing alginate gel fiber B

Following the manufacturing steps shown schematically in Fig. 2, an alginate gel fiber B was prepared under the same conditions as the alginate gel fiber described in Japanese Patent Application Publication No. 2016-77229 (Applicant: National University Corporation, University of Tokyo). Tocilizumab-producing CHO cells were used as the cells. An 0.2% collagen solution (5 mg acidic collagen solution I-PC, pH 3.0, sterile Atelocollag, Koken Co., Ltd. Cat. #IPC-50) containing cells at a concentration of 5×10⁶ cells/ml (1×10⁶ cells per one 25 m fiber) was introduced through the inlet 1 of the microfluidic device 10, while a 1.5 mass% (w/w%) sodium alginate (B-2, Mochida Pharmaceutical Co., Ltd) solution (solvent: medium with composition of Table 3 below) was introduced from the inlet 2, and 100 mM calcium chloride aqueous solution was introduced and injected through the inlet 3 to obtain an alginate gel fiber B with a core layer diameter of about 100 µm, an outer diameter of about 300 µm, and a total length of 25 meters.

### [Example 3] Culturing tocilizumab-producing CHO cells in alginate gel fiber A

The alginate gel fiber A (outer diameter 300 µm×length 25 m, fiber volume (outer part) about 1.8 ml) obtained in Example 1 was placed in a 125 ml triangular polycarbonate flask, and 30 ml of medium with the composition shown in Table 3 was added to impregnate the fiber. Culture was performed for 12 days under shaking at 125 rpm in a 5% CO₂ incubator at 37°C. It was confirmed that an antibody (tocilizumab) was produced by 12 days of culture and released into the liquid outside the fiber. ELISA measurement using human IL-6 Receptor a (Peprotech, Cat. #200-06RC) was used to confirm that the produced antibody was tocilizumab. The results of this test also showed that the produced antibody could pass outside the fiber. Fig. 4 shows a microscope image of the alginate gel fiber after 12 days of culture.

Medium composition: The additives shown in the table below were added to G016 medium to a total volume to 1,000 ml.

**[Table 3]**

| | Sample | Maker | Added amount (ml) | Final concentration |
|---|---|---|---|---|
| Medium | G016 medium | Irvine | 930 | |
| Additives | L-glutamine 200 mM | SIGMA | 40 | 8 mM |
| | Penicillin Streptmycin | Invitrogen | 10 | 1% |
| | Soy hydrolysate UF solution 50X | SIGMA | 20 | 2% |

### [Example 4] Culturing tocilizumab-producing CHO cells in alginate gel fiber B

The alginate gel fiber B (outer diameter 300 µm×length 25 m, fiber volume (outer part) about 1.8 ml) obtained in Example 2 was placed in a 125 ml triangular polycarbonate flask, and 30 ml of medium with the composition shown in Table 3 was added to impregnate the fiber. Culture was performed for 28 days under shaking at 125 rpm in a 5% CO₂ incubator at 37°C. 4.77 mg of the antibody (tocilizumab) were produced by 28 days of culture. ELISA measurement using human IL-6 Receptor a (Peprotech, Cat. #200-06RC) was used to confirm that the produced antibody was tocilizumab. The results of this test also showed that the produced antibody could pass outside the fiber. Fig. 5 shows a microscope image of the alginate gel fiber after 28 days of culture.

### [Industrial Applicability]

Provided is an alginate gel fiber (alginate hollow microfiber) for antibody production comprising antibody-producing cells comprised in a core layer. A method for manufacturing this alginate gel fiber and a method for culturing antibodies using this alginate gel fiber are also provided.

### [Reference Signs List]

- a: Diameter of core layer (hollow part) of alginate gel fiber
- b: Thickness of shell layer of alginate gel fiber
- c: Outer diameter of alginate gel fiber
- 4: Shell layer
- 5: Core layer
- 6: Antibody-producing cells

- 10: Microfluidic device
- 1: Inlet for base material comprising antibody-producing cells 6
- 2: Inlet for sodium alginate solution
- 3: Inlet for calcium chloride solution
- 20: Alginate gel fiber
- 40: Introduction pipe
- 50: Outlet

## Claims

1. An alginate gel fiber formed by covering a core layer comprising antibody-producing cells and a base material with a shell layer comprising an alginate gel having greater mechanical strength than the core layer.

2. The alginate gel fiber according to claim 1, wherein the antibody-producing cells comprised in the core layer are cells selected from the group consisting of the CHO cells, CHO cell substrains, COS cells, Sp2/0 cells, NS0 cells, SP2 cells, PERC6 cells, YB2/0 cells, YE2/0 cells, 1R983F cells, Namalwa cells, Wil-2 cells, Jurkat cells, Vera cells, Molt-4 cells, 293-HEK cells, BHK cells, KGH6 cells, P3X63Ag8.653 cells, C127 cells, JC cells, LA7 cells, ZR-45-30 cells, hTERT cells, NM2C5 cells and UACC-812 cells.

3. The alginate gel fiber according to claim 1 or 2, wherein the base material comprised in the core layer is a base material selected from the group consisting of a collagen solution, a collagen gel, a medium (or culture solution), an alginate solution (such as a sodium alginate solution), an alginate gel and mixtures thereof.

4. The alginate gel fiber according to any one of claims 1 to 3, wherein the raw material of the alginate gel comprised in the shell layer is sodium alginate, and the M/G ratio of the sodium alginate is in the range of from 0.4 to 1.8 or in the range of from 0.1 to 0.4.

5. The alginate gel fiber according to any one of claims 1 to 4, wherein the raw material of the alginate gel comprised in the shell layer is sodium alginate, and the molecular weight (GPC) of the sodium alginate is in the range of from 700,000 to 1,000,000 or in the range of from 800,000 to 1,000,000.

6. The alginate gel fiber according to any one of claims 1 to 5, wherein the raw material of the alginate gel comprised in the shell layer is sodium alginate, and the 1 w/w% viscosity of the sodium alginate is in the range of from 50 to 150 (mPa • s) or in the range of from 70 to 150 (mPa • s).

7. The alginate gel fiber according to any one of claims 1 to 6, wherein the outer diameter of the alginate gel fiber is from 0.2 µm to 2,000 µm, while the core layer of the alginate gel fiber has a diameter of from 0.1 µm to 1,000 µm.

8. A method for manufacturing an alginate gel fiber formed by covering a core layer comprising antibody-producing cells and a base material with a shell layer comprising an alginate gel having greater mechanical strength than the core layer, wherein
the method for manufacturing an alginate gel fiber uses a microfluidic device 10 comprising an introduction pipe 40, an inlet 1 of the introduction pipe 40, an inlet 2 of the introduction pipe 40 located downstream from the inlet 1, an inlet 3 of the introduction pipe 40 located downstream from the inlet 2, and an outlet 50 of the introduction pipe 40 located downstream from the inlet 2, and comprises
(1) a step of forming a first laminar flow of the antibody-producing cells 6 and the base material in the introduction pipe 40 by introducing antibody-producing cells 6 and a base material from the inlet 1 and injecting the same,
(2) a step of forming a second laminar flow of the sodium alginate solution covering the outer circumference of the first laminar flow by introducing a sodium alginate solution from the inlet 2 and injecting the same,
(3) a step of forming a third laminar flow of the solution comprising the divalent metal ion covering the outer circumference of the second laminar flow by introducing a solution comprising a divalent metal ion from the inlet 3 and injecting the same, and
(4) a step of obtaining an alginate gel fiber formed by covering a core layer comprising antibody-producing cells and a base material that are ejected from the outlet 50 with a shell layer comprising an alginate gel.

9. The method for manufacturing an alginate gel fiber according to claim 8, wherein the antibody-producing cells 6 are the cells according to claim 2.

10. The method for manufacturing an alginate gel fiber according to claim 8 or 9, wherein the base material comprised in the core layer is a base material selected from the group consisting of a collagen solution, a collagen gel, a medium (or culture solution), an alginate solution (such as a sodium alginate solution), an alginate gel and mixtures thereof.

11. The method for manufacturing an alginate gel fiber according to any one of claims 8 to 10, wherein the divalent metal ion is an ion selected from the group consisting of calcium ions, magnesium ions, barium ions, strontium ions and zinc ions.

12. The method for manufacturing an alginate gel fiber according to any one of claims 8 to 11, wherein the solution comprising the divalent metal ion is an aqueous solution selected from the group consisting of a calcium chloride aqueous solution, a calcium carbonate aqueous solution and a calcium gluconate aqueous solution.

13. The method for manufacturing an alginate gel fiber according to any one of claims 8 to 12, wherein the temperature during manufacture of the alginate gel fiber is in the range of from 4°C to 25°C.

14. A method for manufacturing an antibody using an alginate gel fiber formed by covering a core layer comprising antibody-producing cells and a base material with a shell layer comprising an alginate gel, wherein an alginate gel fiber according to any one of claims 1 to 7 is placed in a culture vessel, medium is added to impregnate the alginate gel fiber, and shaking culture is performed to produce the antibody.
